# EUROPEAN PATENT APPLICATION

(11) **EP 3 590 461 A1**
(43) Date of publication of application: **08.01.2020**
(21) Application number: 19183673.3
(22) Date of filing: 01.07.2019
(51) Int. Cl.: A61B 90/98, G06K 19/067, G06K 19/077

(54) **METHOD AND APPARATUS RELATED TO FABRICATED WIRELESS TRANSPONDER DEVICES TO BE USED IN MEDICAL PROCEDURES**

(30) Priority: 02.07.2018 US 201862693152 P; 03.06.2019 US 201916429282
(71) Applicant: Covidien LP, Mansfield, MA 02048 (US)
(72) Inventor: AQUINO, Allan G., Longmont, CO Colorado 80503 (US); BRANDT, Kim, Loveland, CO Colorado 80537 (US); BUERSMEYER, Andy, Ft. Collins, CO Colorado 80526 (US); POIRIER, David A., Escondido, CO Colorado 92029 (US)
(74) Representative: Maschio, Antonio

(57) **Abstract**

A wireless transponder device that may be used in medical procedure may be fabricated using semiconductor fabrication techniques. The electrical components of the wireless transponder device may be more resilient when compared to components that are electrically coupled using traditional electrical coupling techniques (e.g., soldering) that may be used for such devices. Such electrical components may be smaller and thereby less noticeable when the wireless transponder is physically coupled or attached to medical devices or components. The semiconductor fabrication process may be used to form a wireless transponder with a metal layer that may be carried on a substrate in which the metal layer includes an inductive portion and a capacitive portion that together may form a resonant circuit that resonates at a desired frequency. The wireless transponder may be enclosed within a pouch that is physically coupled to a medical object that may be used during the medical procedure.

## Description

### Technical Field

The present disclosure generally relates to a wireless transponder device that is used in medical procedures in which the wireless transponder device includes a substrate that carries one or more metal layers with an inductive portion and a capacitive portion.

### BACKGROUND

### Description of the Related Art

It is important to determine whether objects or items associated with a medical or clinical procedure are present or unintentionally retained in a patient's body before completion of a medical or clinical procedure. The medical or clinical procedure may, for example, take the form of a surgery or childbirth delivery. Such objects or items may take a variety of forms used in medical or clinical procedures. For example, the objects or items may take the form of instruments, for instance scalpels, scissors, forceps, hemostats, and/or clamps, which may be reusable after sterilization or alternatively may be single-use disposable objects or items. Also for example, the objects or items may take the form of related accessories and/or disposable objects, for instance disposable surgical sponges, gauzes, and/or absorbent pads. When used in surgery, failure to locate an object or item before closing the patient may require additional surgery, and in some instances may have serious adverse medical consequences. In other medical procedures, such as vaginal childbirth deliveries, failure to remove objects, for instance gauze or absorbent pads, can lead to infections and undesired complications.

Some hospitals have instituted procedures that include checklists or requiring multiple manual counts to be performed to track the use and return of objects or items during surgery. Such a manual approach is inefficient, requiring the time of highly trained personnel, and is prone to error.

Another approach employs wireless transponders that are attached to various objects or items used during surgery, and a wireless interrogation and detection system. Such an approach can employ "dumb" wireless transponders, *i.e.*, wireless communications transponders that do not store and/or transmit any unique identifying information. Dumb wireless transponders have traditionally been employed for electronic article surveillance (EAS) to prevent loss of merchandise at retail locations. Alternatively, such an approach can employ radio frequency identification (RFID) wireless transponders, *i.e.*, wireless communications transponders which do store and return a unique identifier in response to an interrogation signal emitted by an RFID interrogator or RFID reader.

In the approach that employs dumb wireless transponders, an interrogation and detection system includes a transmitter that emits pulsed wireless interrogation signals (*e.g*., radio or microwave frequency) and a detector for detecting wireless response signals returned by the dumb wireless transponders in response to the emitted interrogation signals. Such an automated system detects the presence or absence of dumb wireless transponders, but typically does not detect any unique identifying information. Since no power is required to operate the dumb wireless transponder, such an approach may have better range or better ability to detect objects or items retained within bodily tissue as compared to RFID wireless transponders communicating in similar ranges of wavelength and levels of power, but cannot uniquely identify the dumb wireless transponders.

In the approach that employs RFID wireless transponders, an interrogator or reader includes a transmitter that emits wireless interrogation signals (*e.g*., radio or microwave frequency) and a detector for detecting wireless response signals returned by the RFID wireless transponders in response to the emitted interrogation signals. Such an automated system advantageously detects the unique identifiers of the RFID wireless transponders; however since some of the power in the interrogation signal is required to operate the RFID wireless transponder such an approach may have shorter range or less ability to detect objects or items retained within bodily tissue as compared to dumb wireless transponders communicating in similar ranges of wavelength and levels of power.

Commercial implementation of such an automated system requires that the overall system be cost competitive, highly accurate, and easy to use. In particular, the transponder should be resilient and perform consistently. Conventional transponders use discrete wire wound around a core, such as a ferrite core. Such transponders may be encapsulated in a plastic tube for protection from fluids and mechanical stresses.

### BRIEF SUMMARY

Soldering may be used to electrically couple together components within conventional transponders, but soldering electrical components may result in a weak physical coupling between the components. As such, the soldered joints may become damaged and thereby electrically decoupled, even during ordinary use.

A wireless transponder device to be used in medical procedures may be summarized as including a wireless transponder including a substrate; a first metal layer carried on the substrate, the first metal layer which includes at least part of a first inductive portion and at least part of a first capacitive portion, the first inductive portion and the first capacitive portion electrically coupled together; and a plating layer, the plating layer electrically coupled to the first metal layer.

The wireless transponder device may further include a ferrite layer, the ferrite layer which is magnetically coupled to at least a portion of the first metal layer proximate the first inductive portion of the metal layer. The ferrite layer may be carried by at least the portion of the first metal layer proximate the first inductive portion of the first metal layer.

The wireless transponder device may further include a second metal layer, the second metal layer which is carried by the ferrite layer and which is electrically coupled to the first metal layer by one or more vias, in which the vias electrically couple the second metal layer to the first metal layer. The ferrite layer may include an exterior surface, wherein the first metal layer, the one or more vias, and the second metal layer form a multi-turn coil that traverses along at least a portion of the exterior surface of the ferrite layer. The second metal layer may be the plating layer. The first capacitive portion may include a first electrically capacitive portion and a second electrically capacitive portion in which a dielectric portion separates the first electrically capacitive portion and the second electrically capacitive portion. The first metal layer may be comprised of alumina. The plating layer may be comprised of copper.

The wireless transponder device may further include a pouch, wherein the wireless transponder is enclosed within the pouch.

A method for fabricating a wireless transponder device that is attached to a medical object for use in medical procedures, the wireless transponder device including a substrate, may be summarized as including depositing a first layer on the substrate; placing a mask on the first layer; etching the first layer according to the mask to define a first cavity therein; depositing a first metal layer within the at least a portion of the first cavity, the first metal layer which includes at least part of a first inductive portion and at least part of a first capacitive portion, the first inductive portion and the first capacitive portion which are electrically coupled together; planarizing the first metal layer, the planarizing which removes at least a portion of the first metal layer; and electrically coupling the planarized first metal layer with a plating layer.

The method may further include enclosing the plated, planarized first metal layer within a pouch, at least a portion of the pouch which is physically coupled to the medical object. The depositing of the first metal layer may include depositing using vacuum deposition. The depositing the first metal layer may include depositing a layer of alumina.

The method may further include depositing a ferrite layer, wherein the ferrite layer is magnetically coupled to at least part of the first metal layer proximate the inductive portion. Depositing the ferrite layer may include sputtering ferrite material. The ferrite layer may be deposited on at least a portion of the first metal layer proximate the first inductive portion.

The method may further include depositing a second metal layer on the ferrite layer; and electrically coupling at least a portion of the second metal layer with at least a portion of the first metal layer using one or more vias, wherein the at least portion of the second metal layer, the one or more vias, and the at least portion of the first metal layer form a multi-turn coil that traverses along at least a portion of an exterior surface of the ferrite layer. Electrically coupling the planarized first metal layer may include electrically coupling the planarized first metal layer with copper.

The method may further include depositing a plating layer, the plating layer which is electrically coupled to the first metal layer.

### BRIEF DESCRIPTION OF THE SEVERAL VIEWS OF THE DRAWINGS

In the drawings, identical reference numbers identify similar elements or acts. The sizes and relative positions of elements in the drawings are not necessarily drawn to scale. For example, the shapes of various elements and angles are not drawn to scale, and some of these elements are arbitrarily enlarged and positioned to improve drawing legibility. Further, the particular shapes of the elements as drawn, are not intended to convey any information regarding the actual shape of the particular elements, and have been solely selected for ease of recognition in the drawings.
Figure 1 is a schematic view of a wireless transponder that includes a substrate and a metal layer carried on the substrate in which the metal layer includes an inductive portion and a capacitive portion, according to at least one illustrated implementation.
Figure 2A is a side level view an inductive portion of a wireless transponder that includes a multi-turn coil formed on multiple layers, according to at least one illustrated implementation.
Figure 2B is a top plan view of the multi-turn coil of Figure 2A in which components on lower levels are shown in a dotted-line representation, according to at least one illustrated implementation.
Figure 3 is a top plan view of an inductive portion of a wireless transponder that includes a multi-turn coil fabricated in one layer, according to at least one illustrated implementation.
Figure 4 is a top plan view of a capacitive portion of a wireless transponder that includes a first electrically conductive portion and a second electrically conductive portion separated by a dielectric portion, according to at least one illustrated implementation.
Figure 5A is a side elevation view of a portion of a target that includes a substrate that carries a non-conductive layer, according to at least one illustrated implementation.
Figure 5B is a side elevation view of the portion of the target in which a masking layer is formed on the non-conductive layer, according to at least one illustrated implementation.
Figure 5C is a side elevation view of the portion of the target in which the non-conductive layer has been etched according to the masking layer, according to at least one illustrated implementation.
Figure 5D is a side elevation view of the portion of the target in which the masking layer has been removed, according to at least one illustrated implementation.
Figure 5E is a side elevation view of the portion of the target in which a metal layer is deposited and carried on the substrate, according to at least one illustrated implementation.
Figure 5F is a side elevation view of the portion of the target in which the metal layer has been planarized, according to at least one illustrated implementation.
Figure 5G is a side elevation view of the portion of the target in which a plating layer is deposited on and electrically coupled to the metal layer, according to at least one illustrated implementation.
Figure 6A is an isometric view of a wireless transponder device that may be used in medical procedures in which the wireless transponder device includes a pouch and a wireless transponder, according to at least one illustrated implementation.
Figure 6B is a side elevation view of a cut away of the wireless transponder device of Figure 6A.
Figure 7 is a flow diagram showing a workflow or method of fabricating a wireless transponder device that is attached to a medical object for use in medical procedures, according to at least one illustrated implementation.

### DETAILED DESCRIPTION

In the following description, certain specific details are set forth in order to provide a thorough understanding of various disclosed embodiments. However, one skilled in the relevant art will recognize that embodiments may be practiced without one or more of these specific details, or with other methods, components, materials, etc. In other instances, well-known structures associated with transmitters, receivers, or transceivers and/or medical equipment and medical facilities have not been shown or described in detail to avoid unnecessarily obscuring descriptions of the embodiments.

Unless the context requires otherwise, throughout the specification and claims which follow, the word "comprise" and variations thereof, such as "comprises" and "comprising," are to be construed in an open, inclusive sense, that is as "including, but not limited to."

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

As used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

The headings and Abstract of the Disclosure provided herein are for convenience only and do not interpret the scope or meaning of the embodiments.

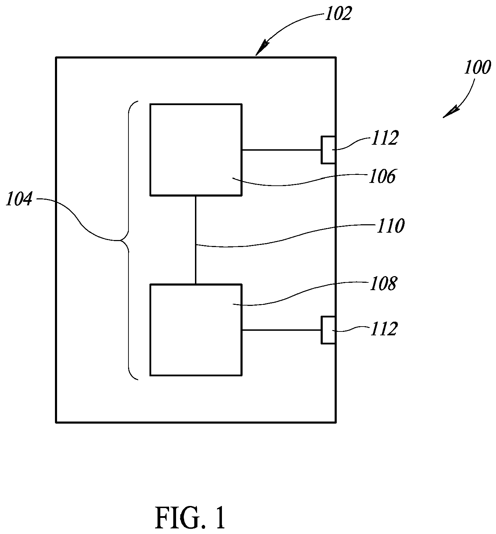

Figure 1 shows a schematic of a wireless transponder 100 that includes a substrate 102 and at least one metal layer 104 carried on the substrate 102 in which the metal layer(s) 104 includes an inductive portion 106 and a capacitive portion 108, according to at least one illustrated implementation. The substrate 102 may be comprised of one or more electrically insulative and/or semiconductor materials such as those that may be used in fabricating integrated circuits. Such materials may include, for example, silicon, silicon dioxide, aluminum dioxide and/or other metal oxides, germanium, gallium nitride, and/or gallium arsenide.

At least one of the metal layers 104 may be carried on the substrate 102. In some implementations, the at least one metal layer 104 may be deposited on the substrate 102 using one or more deposition processes. The deposition processes may include, for example, one or more types of chemical vapor deposition techniques, and/or one or more types of physical vapor deposition techniques, such as sputtering. The metal layer(s) 104 may include one or more inductive portions 106 and one or more capacitive portions 108. In such an implementation, the inductive portion 106 may be electrically coupled to the capacitive portion 108 to thereby form an LC circuit that resonates at a desired frequency. In some implementations (not shown), the metal layer(s) 104 may further include a resistive portion that may be electrically coupled to the inductive portion 106 and the capacitive portion 108 to form an RLC circuit that resonates at a desired frequency.

In some implementations, the metal layer(s) 104 may include one or more electrically conductive traces 110. In some implementations, the electrically conductive traces 110 may be deposited and carried on the substrate 102. The conductive traces 110 may electrically couple one or more of the inductive portions 106, the conductive portion 108, and/or the resistive portion (not shown). The conductive traces 110 may be formed during the same or a different deposition process (e.g., the chemical vapor deposition process and/or the physical vapor deposition process) as the inductive portion 106 and/or the conductive portion 108. By forming and electrically coupling the inductive portion 106 and the conductive portion 108 using a deposition process, the electrical coupling between the inductive portion 106 and the conductive portion 108 may be more resilient than electrical couplings that use, for example, soldering or other similar techniques.

In some implementations, the substrate 102 may include one or more ports 112. Such ports 112 may be used to electrically couple the inductive portion 106 and/or the conductive portion 108 of the metal layer 104 to other electrical components. The ports 112, for example, may be used to electrically couple the components of the metal layer 104 to a component that may be used to enhance the ability of the LC or RLC circuit to resonate at a desired frequency. Such a component may include, for example, a metal structure that may serve as an antenna for the LC circuit carried on the substrate 102.

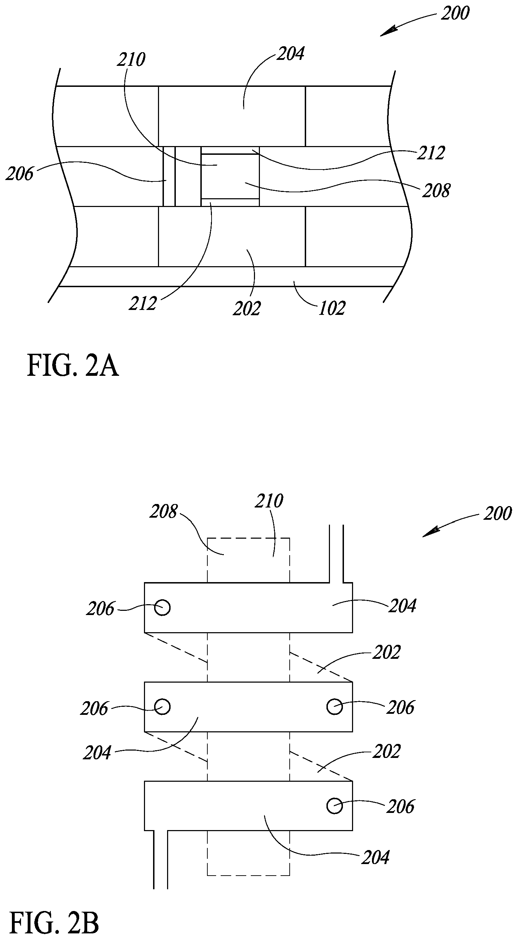

Figures 2A and 2B show an inductive portion 106 of a wireless transponder 100 that includes a multi-turn coil 200 formed on multiple layers, according to at least one illustrated implementation. The multi-turn coil 200 may include, for example, one or more bottom metal layers 202, one or more top metal layers 204, and one or more vias 206. Each bottom metal layer 202 may be aligned with one, two, or more corresponding top metal layers 204. For bottom metal layers 202 aligned with two corresponding top metal layers 204, the corresponding top metal layers 204 may be aligned with opposing ends of the bottom metal layer 202. Each top metal layer 204 may be aligned with one, two, or more corresponding bottom metal layers 202. For top metal layers 204 aligned with two corresponding bottom metal layers 202, the corresponding bottom metal layers 202 may be aligned with opposing ends of the top metal layer 204. Each via 206 may extend between a bottom metal layer 202 and a corresponding top metal layer 204 to electrically couple the bottom metal layer 202 with the corresponding top metal layer 204.

In some implementations, the multi-turn coil 200 may be formed proximate a ferrite layer 208. For example, in some implementations, the ferrite layer is formed between the bottom metal layer 202 and the top metal layer 204. In such implementations, the ferrite layer 208 may be deposited through one or more deposition processes, such as, for example, a sputtering process. The ferrite layer 208 may have an exterior surface 210 around which the multi-turn coil 200 traverses. In some implementations, one or both of the bottom metal layer 202 and/or the top metal layer 204 may be set at a non-right angle compared to a length of the ferrite layer 208. For example, as shown in Figure 2B, the bottom metal layer 202 is arranged at a non-right angle to the ferrite layer 208 such that the multi-turn coil 200 may traverse down a length of the ferrite layer 208. In some implementations, the ferrite layer 208 may be separated from the bottom metal layer 202 and/or the top metal layer 204 through one or more insulating layers, such as insulating layers 212. As such, the ferrite layer 208 may be magnetically coupled to the multi-turn coil 200, and may thereby form an inductor when electric current is flowing through the multi-turn coil 200.

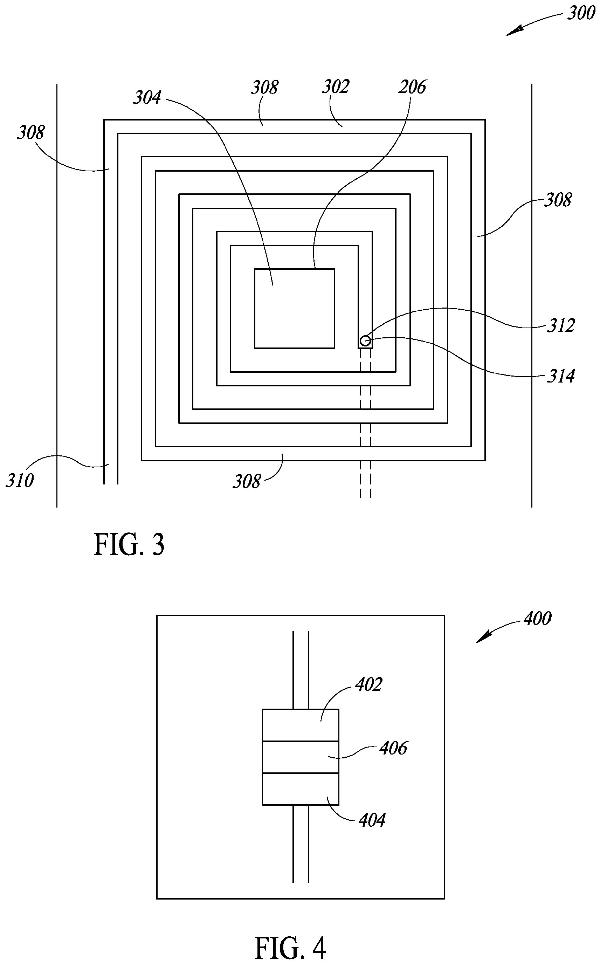

Figure 3 shows an inductive portion 300 of a wireless transponder 100 that includes a multi-turn coil 302 fabricated in one layer, according to at least one illustrated implementation. The multi-turn coil 302 may be formed around a core 304, such as a ferrite core and/or an air core, in which the core 304 has a perimeter 306. The multi-turn coil 302 may include multiple legs 308 (four identified in Figure 3) that may physically couple together to traverse around the perimeter 306 of core 304. The multi-turn coil 302 may include an exterior end 310 and an interior end 312 in which the exterior end 310 is located relatively further away from the core than the interior end 312. The legs 308 may get progressively closer to the core 304 when traversing the physically coupled legs from the exterior end 310 to the interior end 312. Electric current may flow through the multi-turn coil 302 between the exterior end 310 towards the interior end 312. One or both of the exterior end 310 and the interior end 312 may include a via 314 to electrically couple the multi-turn coil 302 with a component or trace on another layer of the wireless transponder 100. In some implementations, such as those involving a ferrite core, the multiple legs 308 of the multi-turn coil 302 may magnetically couple with the ferrite core when electric current is flowing through the multi-turn coil 302 to thereby act as an inductor when electric current is flowing through the multi-turn coil 302.

Figure 4 shows a capacitive portion 400 of a wireless transponder 100 that includes a first electrically conductive portion 402 and a second electrically conductive portion 404 separated by a dielectric portion 406, according to at least one illustrated implementation. The electrically capacitive portions 402, 404 may be comprised of one or more conductive materials, such as alumina and/or copper that may be deposited by one or more methods onto the wireless transponder 100. The dielectric portion 406 may be comprised of one or more dielectric materials that electrically insulate the first electrically conductive portion 402 from the second electrically conductive portion 404. Such dielectric materials may include, for example, silicon oxide and/or other oxides. In some implementations, such as those shown in Figure 4, the capacitive portion 400 may be formed in a single layer. In some implementations, the capacitive portion may be formed in multiple layers of the wireless transponder 100.

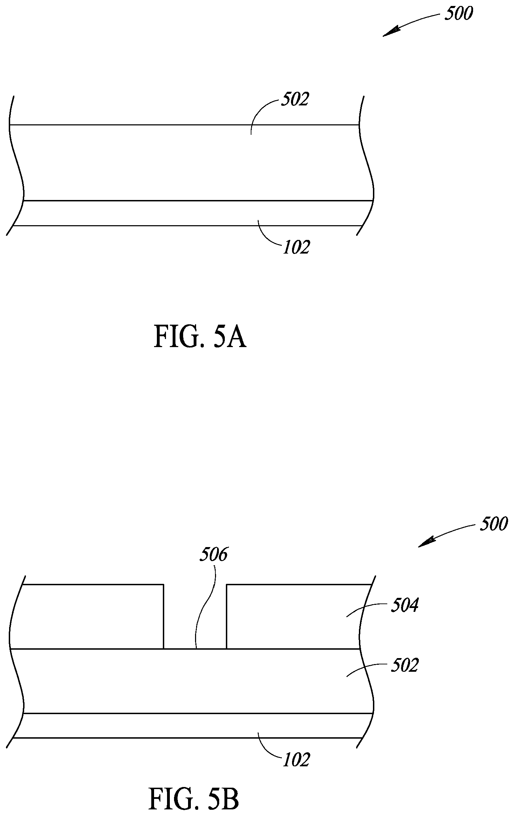

Figure 5A shows a portion of a target 500 that includes a substrate 102 that carries a first layer 502, such as a non-conductive layer, according to at least one illustrated implementation. The substrate may be comprised of one or more electrically insulative and/or semiconductor materials such as those that may be used in fabricating integrated circuits. Such materials may include, for example, silicon, silicon dioxide, aluminum dioxide and/or other metal oxides, germanium, gallium nitride, and/or gallium arsenide. The first layer 502, for example, may be an oxide layer that is formed on the surface of the substrate 102. Such an oxide layer may be formed using chemical processes. In some implementations, the first layer 502 may be deposited on the substrate 102 using one or more techniques. For example, in some implementations, the first layer 502 may be deposited on the substrate 102 using vacuum deposition. In some implementations, the first layer 502 may be comprised of one or more conductive materials, such as alumina, that may be deposited on and/or applied to the substrate 102, using, for example, a chemical vapor deposition and/or physical vapor deposition process.

Figure 5B shows the portion of the target 500 in which a masking layer 504 is formed on the first layer 502, according to at least one illustrated implementation. The masking layer 504 may be formed by depositing a photoresist layer on the first layer 502 and exposing the photoresist layer to a masked pattern of ultraviolet light. In some implementations involving a negative photoresist, the portions of the photoresist layer exposed to the ultraviolet light may become hardened such that applying a solvent to the photoresist layer will dissolve the non-hardened portion, thereby forming the masking layer 504. In some implementations involving a positive photoresist, the portions of the photoresist layer exposed to the ultraviolet light may degrade such that applying a solvent to the photoresist layer will dissolve the degraded portion, thereby forming the masking layer 504. The masking layer 504 may leave an exposed portion 506 of the first layer 502.

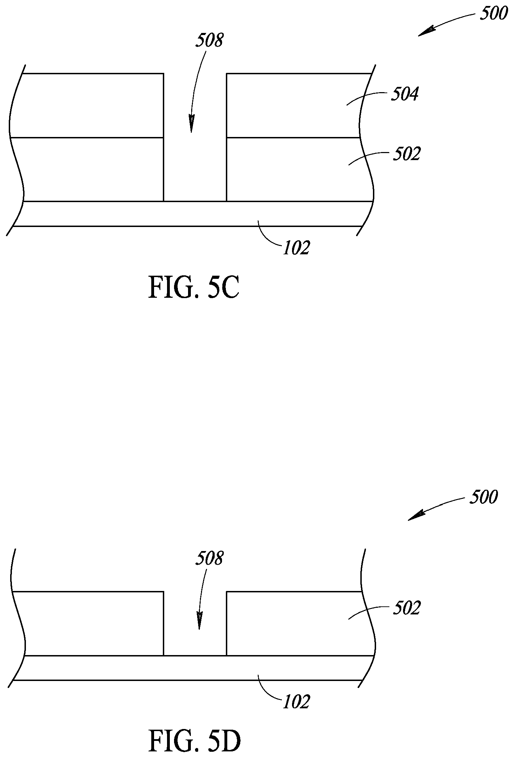

Figure 5C shows the portion of the target 500 in which the first layer 502 has been etched according to the masking layer 504, according to at least one illustrated implementation. The etching of the first layer 502 may be performed using various processes. For example, in some implementations, the etching may be performed using a wet etching process in which the substrate 102, the first layer 502, and the masking layer 504 are immersed in a bath of etchant that may react with and etch the exposed portion 506 of the first layer 502. In some implementations, a plasma etching process may be used to etch the exposed portion 506 of the first layer 502. The removal of the exposed portion 506 of the first layer 502 may be used to form a cavity 508 within the first layer 502. Such a cavity 508 may be used to form electrical components and/or to form electrical traces that may be used to electrically couple various electrical components.

Figure 5D shows the portion of the target 500 in which the masking layer 504 has been removed, according to at least one illustrated implementation. The masking layer 504 may be removed by applying a solution to the masking layer 504 that reacts with and dissolves the masking layer 504. The process thereby leaves the first layer 502 and the cavity 508.

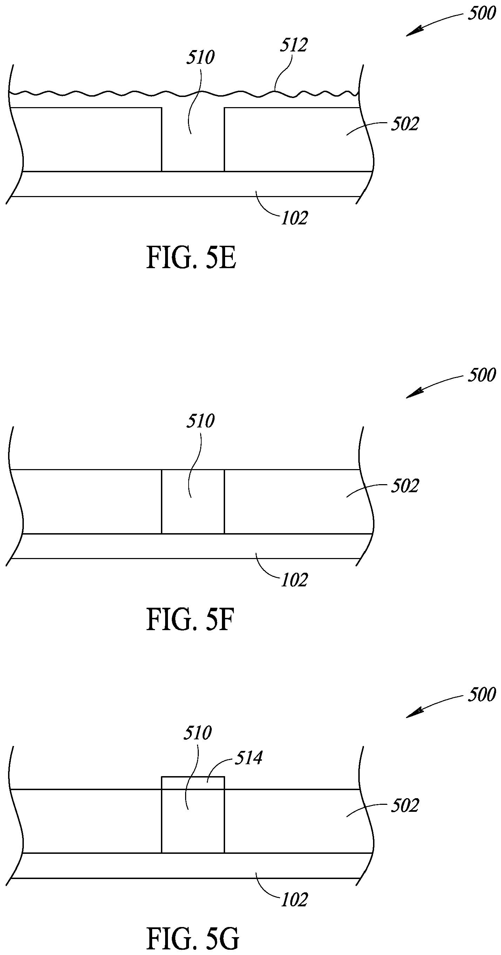

Figure 5E shows the portion of the target 500 in which a layer 510 is deposited and carried on the substrate 102, according to at least one illustrated implementation. In some implementations, the layer 510 may be deposited by one or more techniques. For example, in some implementations, the layer 510 may be deposited using one or more of chemical vapor deposition, physical vapor deposition, electrochemical deposition, molecular beam epitaxy, or some other type of technique in which a metal is deposited within the cavity 508 and carried by the substrate 102. For example, in some implementations, the layer 510 may be deposited using a sputtering technique. Such a layer 510 may include an exposed surface 512 that may be opposite the substrate 102. In some implementations, the layer 510 may be a metal layer. In some implementations, the layer 510 may be comprised of a ferrite material and/or a dielectric material.

Figure 5F shows the portion of the target 500 in which the layer 510 has been planarized, according to at least one illustrated implementation. In some implementations, the layer 510 may be planarized using, for example, a chemical-mechanical planarization process. In such an implementation, a chemical slurry may be applied to some or all of an exposed surface 512 of the layer 510, and a polishing pad may be physically applied to the surface of the layer 510 to remove a portion of the exposed surface 512 of the layer 510. In some implementations, the portion of the layer 510 that is carried by the first layer 502 may be removed from the target 500. As such, only the portion of the layer 510 included within the cavity 508 may remain on the target 500. In some implementations, the layer 510 may be doped with one more types of doping components that may be used to modify and/or control the electrical properties of the layer 510. As such, for example, the doped layer 510 may be used to form an inductive portion and/or a capacitive portion on the target 500.

Figure 5G shows the target 500 in which a plating layer 514 is deposited on and electrically coupled to the layer 510, according to at least one illustrated implementation. In some implementations, the plating layer 514 may be comprised of copper. As such, the plating layer 514 may be used to electrically couple different components within the target.

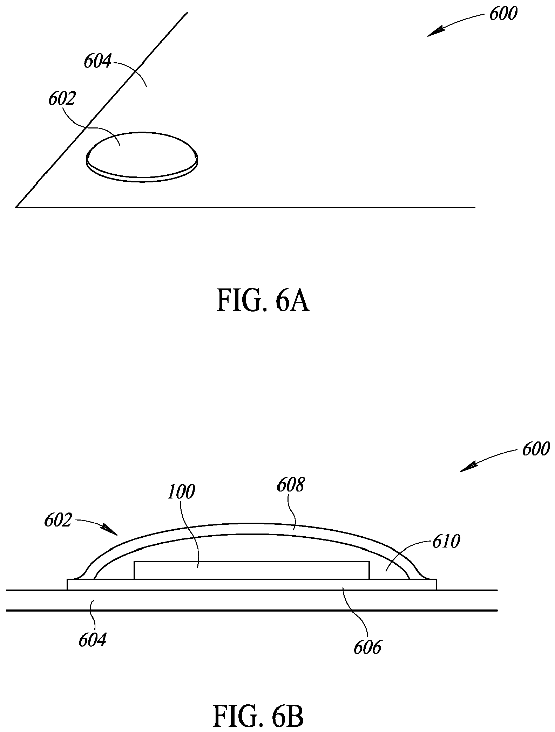

Figures 6A and 6B show a wireless transponder device 600 that may be used in medical procedures in which the wireless transponder device 600 includes a pouch 602 and a wireless transponder 100 within the pouch (Figure 6B), according to at least one illustrated implementation. In some implementations, the pouch 602 may be attached to a medical object 604 that may be used in a medical procedure. Such a medical object 604 may include various types of medical supplies or accessories such as a medical sponge and/or gauze. Such objects may be denominated as disposable objects, or disposable medical objects, or disposable medical procedure objects. Such a pouch 602 may provide protection for the enclosed wireless transponder 100 against ingress by fluid or other debris, such as may be encountered by medical sponge and/or gauze during a medical procedure. In some implementations, the pouch 602 may be resilient to the various cleaning procedures (e.g., chemical and/or UV processes) that may be used to clean medical supplies or accessories.

In some implementations, the pouch 602 may include a base 606, which may be adjacent the medical object 604, and a cover 608 such that the wireless transponder 100 is enclosed by the base 606 and the cover 608 within a cavity 610. Sealing of the wireless transponder 100 within the cavity 610 between the base 606 and the cover 608 may be done by a variety of sealing methods including heat sealing. A type of heat sealing method that may be used is RF welding. Thus, in one embodiment, the wireless transponder 100 may be RF welded within the cavity 610 between the base 606 and the cover 608. In some embodiments, formation of the pouch 602 (i.e., sealing the wireless transponder 100 between the base 606 and the cover 608) may be done in advance. In some implementations, the cover 608 may be physically coupled directly to the medical object 604 to form the pouch 602 and cavity 610 for enclosing the wireless transponder 100. In some implementations, the base 606 and the cover 608 may be comprised of a single piece of material that has been folded over to form the cavity 610. Further implementations of a pouch that may be used to enclose a wireless transponder 100 are disclosed in one or more of U.S. Patent Applicant Nos. 12/606,686; 14/247,960; and 15/708,048; and U.S. Provisional Patent Application No. 61/109,142, all of which are incorporated herein by reference.

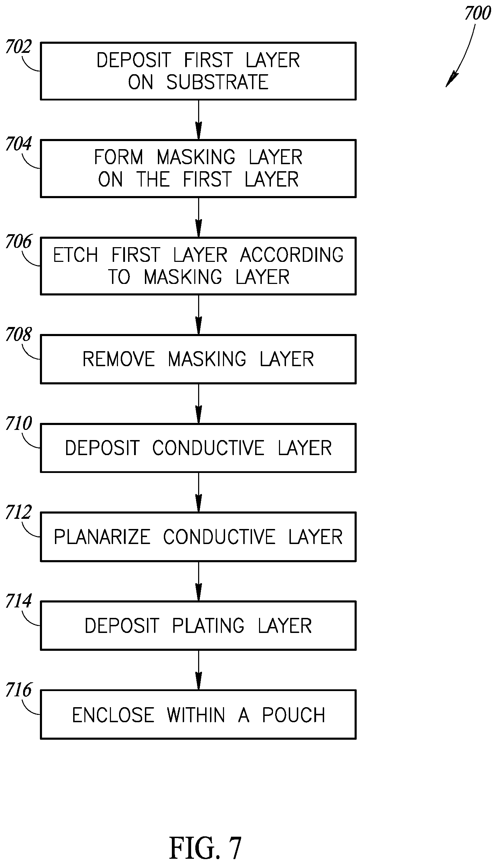

Figure 7 shows a method 700 of fabricating a wireless transponder device 100 that may be attached to a medical object 604 for use in medical procedures, according to at least one illustrated implementation.

The method 700 starts at 702 in which a first layer 502, such a non-conductive layer, is deposited on the substrate 102. The substrate 102 may be comprised of one or more electrically insulative and/or semiconductor materials such as those that may be used in fabricating integrated circuits. Such materials may include, for example, silicon, silicon dioxide, aluminum dioxide and/or other metal oxides, germanium, gallium nitride, and/or gallium arsenide. The first layer 502, for example, may be an oxide layer that is formed on the surface of the substrate 102. Such an oxide layer may be formed using chemical processes. In some implementations, the first layer 502 may be comprised of conductive material, such as alumina, that may be deposited on and/or applied to the substrate 102. In some implementations, the first layer 502 may be deposited on the substrate 102 using one or more techniques. For example, in some implementations, the first layer 502 may be deposited on the substrate 102 using vacuum deposition.

At 704, the masking layer 504 is formed on the first layer 502. The masking layer 504 may be formed by depositing a photoresist layer on the first layer 502 and exposing the photoresist layer to a masked pattern of ultraviolet light. In some implementations involving a negative photoresist, the portions of the photoresist layer exposed to the ultraviolet light may become hardened such that applying a solvent to the photoresist layer will dissolve the non-hardened portion, thereby forming the masking layer 504. In some implementations involving a positive photoresist, the portions of the photoresist layer exposed to the ultraviolet light may degrade such that applying a solvent to the photoresist layer will dissolve the degraded portion, thereby forming the masking layer 504. The masking layer 504 may leave an exposed portion 506 of the first layer 502.

At 706, the first layer 502 is etched according to the masking layer 504. The etching of the first layer 502 may be performed using various processes. For example, in some implementations, the etching may be performed using a wet etching process in which the substrate 102, the first layer 502, and the masking layer 504 are immersed in a bath of etchant that may react with and etch the exposed portion 506 of the first layer 502. In some implementations, a plasma etching process may be used to etch the exposed portion 506 of the first layer 502. The removal of the exposed portion 506 of the first layer 502 may be used to form a cavity 508 within the first layer 502. Such a cavity 508 may be used to form electrical components and/or to form electrical traces that may be used to electrically couple various electrical components.

At 708, the masking layer 504 has been removed. The masking layer 504 may be removed by applying a solution to the masking layer 504 that reacts with and dissolves the masking layer 504. The process thereby leaves the first layer 502 and the cavity 508.

At 710, the layer 510 is deposited and carried on the substrate 102. In some implementations, the layer 510 may be deposited by one or more techniques. For example, in some implementations, the layer 510 may be deposited using one or more of chemical vapor deposition, physical vapor deposition, electrochemical deposition, molecular beam epitaxy, or some other type of technique in which a metal is deposited within the cavity 508 and carried by the substrate 102. For example, in some implementations, the layer 510 may be deposited using a sputtering technique. Such a layer 510 may include an exposed surface 512 that may be opposite the substrate 102. In some implementations, the layer 510 may be a conductive layer, such as a metal layer. In some implementations, the layer 510 may be comprised of a ferrite material and/or a dielectric material. In some implementations, the layer 510 may be an insulative layer comprised, for example, of dielectric material.

At 712, the layer 510 is planarized. In some implementations, the layer 510 may be planarized using, for example, a chemical-mechanical planarization process. In such an implementation, a chemical slurry may be applied to some or all of an exposed surface 512 of the layer 510, and a polishing pad may be physically applied to the surface of the layer 510 to remove a portion of the exposed surface 512 of the layer 510. In some implementations, the portion of the layer 510 that is carried by the first layer 502 may be removed from the target 500. As such, only the portion of the layer 510 included within the cavity 508 may remain on the target 500. In some implementations, the layer 510 may be doped with one more types of doping components that may be used to modify and/or control the electrical properties of the layer 510. As such, for example, the doped layer 510 may be used to form an inductive portion and/or a capacitive portion on the target 500.

At 714, a plating layer 514 is deposited on and electrically coupled to the layer 510. In some implementations, the plating layer 514 may be comprised of copper. As such, the plating layer 514 may be used to electrically couple different components within the target 500. Such electrical coupling of the various components may be used to thereby form the wireless transponder 100.

At 716, wireless transponder 100 may be placed within a pouch 602. In such implementations, the pouch 602 may be attached to a medical object 604 that may be used in a medical procedure. Such a medical object 604 may include various types of medical supplies or accessories such as a medical sponge and/or gauze. Such objects may be denominated as disposable objects, or disposable medical objects, or disposable medical procedure objects. Such a pouch 602 may provide protection for the enclosed wireless transponder 100 against ingress by fluid or other debris, such as may be encountered by medical sponge and/or gauze during a medical procedure. In some implementations, the pouch 602 may be resilient to the various cleaning procedures (*e.g*., chemical and/or UV processes) that may be used to clean medical supplies or accessories. Because the various electrical components of the wireless transponder 100 are formed via a semiconductor deposition process, the electrical coupling may be more resilient when compared, for example, to traditional electrical coupling techniques (*e.g*., soldering) that may be used for such devices. The semiconductor deposition process may also be used to form electrical components that are smaller and thereby less noticeable when the wireless transponder 100 is physically coupled or attached to medical devices or components.

While generally discussed in terms of a passive wireless transponder, which requires an interrogation signal to derive electrical energy to power operation, for example to backscatter a response signal, such is not necessary to all implementations. For example, some implementations can employ an active transponder, with an onboard, consumable power source (*e.g*., chemical battery), which can emit signals from time-to-time (*e.g.*, periodically) without any external stimulus (*e.g.*, interrogation signals). Such implementations are of course subject to the power source being capable of operating over long times, even if the object to which the active wireless transponder is attached is not put into service for several years. Thus, most implementations will employ passive wireless transponders, and thus employ interrogation signals.

Also for instance, the foregoing detailed description has set forth various embodiments of the devices and/or processes via the use of block diagrams, schematics, and examples. Insofar as such block diagrams, schematics, and examples contain one or more functions and/or operations, it will be understood by those skilled in the art that each function and/or operation within such block diagrams, flowcharts, or examples can be implemented, individually and/or collectively, by a wide range of hardware, software, firmware, or virtually any combination thereof. In one embodiment, the present subject matter may be implemented via Application Specific Integrated Circuits (ASICs). However, those skilled in the art will recognize that the embodiments disclosed herein, in whole or in part, can be equivalently implemented in standard integrated circuits, as one or more computer programs running on one or more computers (*e.g*., as one or more programs running on one or more computer systems), as one or more programs running on one or more controllers (*e.g*., microcontrollers) as one or more programs running on one or more processors (*e.g*., microprocessors), as firmware, or as virtually any combination thereof, and that designing the circuitry and/or writing the code for the software and or firmware would be well within the skill of one of ordinary skill in the art in light of this disclosure.

Various exemplary methods or processes are described. It is noted that these exemplary methods or processes may include additional acts and/or may omit some acts. In some implementations, the acts of the various exemplary methods or processes may be performed in a different order and/or some acts may be executed or performed concurrently.

In addition, those skilled in the art will appreciate that the mechanisms of taught herein are capable of being distributed as a program product in a variety of forms, and that an illustrative embodiment applies equally regardless of the particular type of physical signal bearing media used to actually carry out the distribution. Examples of signal bearing media include, but are not limited to, the following: recordable type media such as floppy disks, hard disk drives, CD ROMs, digital tape, and computer memory.

The various embodiments described above can be combined to provide further embodiments. To the extent not inconsistent with the teachings herein, all U.S. patents, U.S. patent application publications, U.S. patent applications, foreign patents, foreign patent applications and non-patent publications commonly owned with this patent application and referred to in this specification and/or listed in the Application Data Sheet including: U.S. Patent No. 6,026,818, issued February 22, 2000; U.S. Patent Publication No. US 2004/0250819, published December 16, 2004; U.S. Patent 8,710,957, issued April 29, 2014; U.S. Patent 7,898,420, issued March 1, 2011; U.S. Patent 7,696,877, issued April 13, 2010; U.S. Patent 8,358,212, issued January 22, 2013; U.S. Patent 8,111,162, issued February 7, 2012; U.S. Patent 8,354,931, issued January 15, 2013; U.S. Patent Publication No. US 2010/0108079, published May 6, 2010; U.S. Patent Publication No. US 2010/0109848, published May 6, 2010; U.S. Patent Publication No. US 2011/0004276, published January 6, 2011; U.S. Patent Publication No. US 2011/0181394, published July 28, 2011; U.S. Patent Publication No. US 2013/0016021, published January 17, 2013; PCT Patent Publication No. WO 2015/152975, published October 8, 2015; U.S. Provisional patent application Serial No. 62/143,726 filed April 6, 2015; U.S. Provisional patent application Serial No. 62/182,294 filed June 19, 2015; U.S. Provisional patent application Serial No. 62/164,612 filed May 20, 2015; U.S. Non-Provisional Patent Application No. 14/523,089 filed October 24, 2014; U.S. Non-Provisional Patent Application No. 14/327,208 filed July 9, 2014; U.S. Non-Provisional Patent Application No. 15/003,515 filed January 21, 2016; U.S. Non-Provisional Patent Application No. 15/003,524 filed January 21, 2016; U.S. Non-Provisional Patent Application No. 15/052,125 filed February 24, 2016; U.S. Non-Provisional Patent Application No. 15/053,965 filed February 25, 2016; U.S. Provisional patent application Serial No. 62/360,864 filed July 11, 2016 and entitled "METHOD AND APPARATUS TO ACCOUNT FOR TRANSPONDER TAGGED OBJECTS USED DURING CLINICAL PROCEDURES, EMPLOYING A SHIELDED RECEPTACLE"; U.S. Provisional patent application Serial No. 62/360,866 filed July 11, 2016 and entitled "METHOD AND APPARATUS TO ACCOUNT FOR TRANSPONDER TAGGED OBJECTS USED DURING CLINICAL PROCEDURES EMPLOYING A SHIELDED RECEPTACLE WITH ANTENNA"; and U.S. Provisional patent application Serial No. 62/360,868 filed July 11, 2016 and entitled "METHOD AND APPARATUS TO ACCOUNT FOR TRANSPONDER TAGGED OBJECTS USED DURING CLINICAL PROCEDURES, FOR EXAMPLE INCLUDING COUNT IN AND/OR COUNT OUT AND PRESENCE DETECTION", are each incorporated herein by reference, in their entirety. Aspects of the embodiments can be modified, if necessary, to employ systems, circuits and concepts of the various patents, applications and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

The invention may be described by reference to the following numbered paragraphs:-
1. A wireless transponder device to be used in medical procedures, the wireless transponder device comprising:
   a wireless transponder comprising:
   a substrate;
   a first metal layer carried on the substrate, the first metal layer which includes at least part of a first inductive portion and at least part of a first capacitive portion, the first inductive portion and the first capacitive portion electrically coupled together; and
   a plating layer, the plating layer electrically coupled to the first metal layer.
2. The wireless transponder device of paragraph 1, further comprising:
   a ferrite layer, the ferrite layer which is magnetically coupled to at least a portion of the first metal layer proximate the first inductive portion of the metal layer.
3. The wireless transponder device of paragraph 2 wherein the ferrite layer is carried by at least the portion of the first metal layer proximate the first inductive portion of the first metal layer.
4. The wireless transponder device of paragraph 3, further comprising:
   a second metal layer, the second metal layer which is carried by the ferrite layer and which is electrically coupled to the first metal layer by one or more vias, in which the vias electrically couple the second metal layer to the first metal layer.
5. The wireless transponder device of paragraph 4 wherein the ferrite layer includes an exterior surface, and wherein the first metal layer, the one or more vias, and the second metal layer form a multi-turn coil that traverses along at least a portion of the exterior surface of the ferrite layer.
6. The wireless transponder device of paragraph 4 wherein the second metal layer is the plating layer.
7. The wireless transponder device of paragraph 1 wherein the first capacitive portion includes a first electrically capacitive portion and a second electrically capacitive portion in which a dielectric portion separates the first electrically capacitive portion and the second electrically capacitive portion.
8. The wireless transponder device of paragraph 1 wherein the first metal layer is comprised of alumina.
9. The wireless transponder device of paragraph 1 wherein the plating layer is comprised of copper.
10. The wireless transponder device of paragraph 1, further comprising:
   a pouch, wherein the wireless transponder is enclosed within the pouch.
11. A method for fabricating a wireless transponder device that is attached to a medical object for use in medical procedures, the wireless transponder device including a substrate, the method comprising:
   depositing a first layer on the substrate;
   placing a mask on the first layer;
   etching the first layer according to the mask to define a first cavity therein;
   depositing a first metal layer within the at least a portion of the first cavity, the first metal layer which includes at least part of a first inductive portion and at least part of a first capacitive portion, the first inductive portion and the first capacitive portion which are electrically coupled together;
   planarizing the first metal layer, the planarizing which removes at least a portion of the first metal layer; and
   electrically coupling the planarized first metal layer with a plating layer.
12. The method of paragraph 11, further comprising:
   enclosing the plated, planarized first metal layer within a pouch, at least a portion of the pouch which is physically coupled to the medical object.
13. The method of paragraph 11 wherein the depositing of the first metal layer includes depositing using vacuum deposition.
14. The method of paragraph 11 wherein the depositing the first metal layer includes depositing a layer of alumina.
15. The method of paragraph 11, further comprising:
   depositing a ferrite layer, wherein the ferrite layer is magnetically coupled to at least part of the first metal layer proximate the inductive portion.
16. The method of paragraph 15 wherein depositing the ferrite layer comprises sputtering ferrite material.
17. The method of paragraph 15 wherein the ferrite layer is deposited on at least a portion of the first metal layer proximate the first inductive portion.
18. The method of paragraph 17, further comprising:
   depositing a second metal layer on the ferrite layer; and
   electrically coupling at least a portion of the second metal layer with at least a portion of the first metal layer using one or more vias, wherein the at least portion of the second metal layer, the one or more vias, and the at least portion of the first metal layer form a multi-turn coil that traverses along at least a portion of an exterior surface of the ferrite layer.
19. The method of paragraph 11 wherein electrically coupling the planarized first metal layer comprises electrically coupling the planarized first metal layer with copper.
20. The method of paragraph 11, further comprising:
   depositing a plating layer, the plating layer which is electrically coupled to the first metal layer.

## Claims

1. A wireless transponder device to be used in medical procedures, the wireless transponder device comprising:
a wireless transponder comprising:
a substrate;
a first metal layer carried on the substrate, the first metal layer which includes at least part of a first inductive portion and at least part of a first capacitive portion, the first inductive portion and the first capacitive portion electrically coupled together; and
a plating layer, the plating layer electrically coupled to the first metal layer.

2. The wireless transponder device of claim 1, further comprising:
a ferrite layer, the ferrite layer which is magnetically coupled to at least a portion of the first metal layer proximate the first inductive portion of the metal layer.

3. The wireless transponder device of claim 2 wherein the ferrite layer is carried by at least the portion of the first metal layer proximate the first inductive portion of the first metal layer.

4. The wireless transponder device of claim 3, further comprising:
a second metal layer, the second metal layer which is carried by the ferrite layer and which is electrically coupled to the first metal layer by one or more vias, in which the vias electrically couple the second metal layer to the first metal layer.

5. The wireless transponder device of claim 4 wherein the ferrite layer includes an exterior surface, and wherein the first metal layer, the one or more vias, and the second metal layer form a multi-turn coil that traverses along at least a portion of the exterior surface of the ferrite layer and/or wherein the second metal layer is the plating layer.

6. The wireless transponder device of any preceding claim wherein the first capacitive portion includes a first electrically capacitive portion and a second electrically capacitive portion in which a dielectric portion separates the first electrically capacitive portion and the second electrically capacitive portion wherein the first metal layer is comprised of alumina and/or wherein the plating layer is comprised of copper.

7. The wireless transponder device of any preceding claim, further comprising:
a pouch, wherein the wireless transponder is enclosed within the pouch.

8. A method for fabricating a wireless transponder device that is attached to a medical object for use in medical procedures, the wireless transponder device including a substrate, the method comprising:
depositing a first layer on the substrate;
placing a mask on the first layer;
etching the first layer according to the mask to define a first cavity therein;
depositing a first metal layer within the at least a portion of the first cavity, the first metal layer which includes at least part of a first inductive portion and at least part of a first capacitive portion, the first inductive portion and the first capacitive portion which are electrically coupled together;
planarizing the first metal layer, the planarizing which removes at least a portion of the first metal layer; and
electrically coupling the planarized first metal layer with a plating layer.

9. The method of claim 8, further comprising:
enclosing the plated, planarized first metal layer within a pouch, at least a portion of the pouch which is physically coupled to the medical object.

10. The method of claim 8 or claim 9 wherein the depositing of the first metal layer includes depositing using vacuum deposition; preferably wherein the depositing the first metal layer includes depositing a layer of alumina.

11. The method of any of claims 8 to 10, further comprising:
depositing a ferrite layer, wherein the ferrite layer is magnetically coupled to at least part of the first metal layer proximate the inductive portion.

12. The method of claim 11 wherein depositing the ferrite layer comprises sputtering ferrite material; preferably wherein the ferrite layer is deposited on at least a portion of the first metal layer proximate the first inductive portion.

13. The method of claim 12, further comprising:
depositing a second metal layer on the ferrite layer; and
electrically coupling at least a portion of the second metal layer with at least a portion of the first metal layer using one or more vias, wherein the at least portion of the second metal layer, the one or more vias, and the at least portion of the first metal layer form a multi-turn coil that traverses along at least a portion of an exterior surface of the ferrite layer.

14. The method of any of claims 8 to 13 wherein electrically coupling the planarized first metal layer comprises electrically coupling the planarized first metal layer with copper.

15. The method of any of claims 8 to 13, further comprising:
depositing a plating layer, the plating layer which is electrically coupled to the first metal layer.
